# EUROPÄISCHE PATENTSCHRIFT

(11) **EP 4 319 661 B1**
(45) Veröffentlichungstag und Bekanntmachung des Hinweises auf die Patenterteilung: **25.12.2024**
(21) Anmeldenummer: 22717206.1
(22) Anmeldetag: 06.04.2022
(51) Int. Cl.: A61B 17/28

(54) **SCHRAUBWERKZEUG SOWIE MONTAGEVERFAHREN MIT SCHRAUBWERKZEUG**
SCREWDRIVING TOOL AND ASSEMBLY METHOD USING A SCREWDRIVING TOOL
OUTIL DE VISSAGE ET PROCÉDÉ D'ASSEMBLAGE UTILISANT UN OUTIL DE VISSAGE

(30) Priorität: 08.04.2021 DE 102021108716
(43) Veröffentlichungstag der Anmeldung: 14.02.2024
(73) Patentinhaber: Aesculap AG, 78532 Tuttlingen (DE)
(72) Erfinder: BARTHELMES, Sven, 78576 Emmingen-Liptingen (DE); MORALES, Pedro, 78532 Tuttlingen (DE)
(74) Vertreter: Winter, Brandl - Partnerschaft mbB
(86) Internationale Anmeldenummer: PCT/EP2022/059087
(87) Internationale Veröffentlichungsnummer: WO 2022/218770

(56) Entgegenhaltungen:
- US-A1- 2007 157 736

## Beschreibung

### Technisches Gebiet

Die vorliegende Offenbarung betrifft ein Schraubwerkzeug zum Übertragen eines Drehmoments auf eine Schraube. Zudem betrifft die vorliegende Offenbarung ein Montageverfahren mit einem, vorzugsweise diesem, Schraubwerkzeug.

### Hintergrund der Erfindung

Zum Eindrehen einer Schraube in ein Innengewinde, etwa um dadurch zwei Branchen eines medizinischen Instruments fest miteinander oder insbesondere zueinander relativbeweglich, beispielsweise verschwenkbar zu verbinden, ist es erforderlich, ein Drehmoment auf die Schraube aufzubringen. Dieses Drehmoment kann insbesondere durch ein Schraubwerkzeug form- und/oder kraftschlüssig (d.h. reibschlüssig) auf die Schraube übertragen werden. Dabei muss das durch das Schraubwerkzeug auf die Schraube übertragene Drehmoment eine zwischen der Schraube und dem Innengewinde wirkende Gewindereibkraft überwinden.

Ein solches Schraubwerkzeug ist beispielsweise aus der US 2009 / 0 229 846 A1 bekannt. Zudem ist ein Montageverfahren zum Einschrauben einer Schraube beispielsweise aus der EP 2 873 381 A1 bekannt.

Zur kraftschlüssigen Drehmomentübertragung wird die Schraube mit einem Axialdruck/einer axialen Druckkraft durch das Schraubwerkzeug beaufschlagt, so dass eine zwischen dem Schraubwerkzeug und der Schraube wirkenden Flächenreibkraft entsteht und bei Drehung des Schraubwerkzeugs ein aus der Flächenreibkraft resultierendes Reibmoment auf die Schraube übertragen wird. Ist das Reibmoment zwischen Schraubwerkzeug und Schraube kleiner als ein durch die Gewindereibkraft resultierendes Moment, rutscht das Schraubwerkzeug durch und die Schraube wird nicht eingeschraubt. Dabei kann es zum Abrutschen des Schraubwerkzeugs und zu Beschädigungen an einer Oberfläche der Schraube oder eines das Innengewinde aufweisenden Bauteils kommen.

Je höher der durch das Schraubwerkzeug auf die Schraube aufgebrachte Axialdruck ist, desto größer sind die Flächenreibkraft und das resultierende, von dem Schraubwerkzeug an die Schraube übertragbare Reibmoment. Da jedoch der auf die Schraube aufgebrachte Axialdruck auch auf das Innengewinde wirkt und somit die Gewindereibkraft mit dem steigenden Axialdruck zunimmt, kann der Axialdruck nicht einfach beliebig erhöht werden, um das reibschlüssig von dem Schraubwerkzeug an die Schraube übertragbare Drehmoment zu erhöhen.

### Zusammenfassung der Offenbarung

Diese dem Anmeldungsgegenstand zugrundeliegende Aufgabe wird durch die im Ansprüche 1 und 9 angegebenen Merkmale gelöst, weitere Ausführungsformen sind in den Unteransprüchen aufgeführt. Es ist also die Aufgabe der vorliegenden Offenbarung, ein Schraubwerkzeug bereitzustellen, mit dem eine Schraube besonders einfach und zuverlässig eingeschraubt werden kann. Zudem soll ein geeignetes Montageverfahren mit einem, vorzugsweise diesem, Schraubwerkzeug zur Verfügung gestellt werden.

Die Aufgabe der vorliegenden Offenbarung wird durch ein Schraubwerkzeug mit den Merkmalen des Patentanspruchs 1 sowie durch ein Montageverfahren mit den Merkmalen des nebengeordneten Patentanspruchs gelöst. Vorteilhafte Weiterbildungen sind Gegenstand der Unteransprüche.

Genauer gesagt hat das Schraubwerkzeug einen ersten Werkzeugschaft, vorzugsweise mit einem großen Schaftdurchmesser, sowie einen zweiten Werkzeugschaft, vorzugsweise mit einem kleinen Schaftdurchmesser. Dabei wird unter dem großen bzw. kleinen Schaftdurchmesser verstanden, dass der Schaftdurchmesser des ersten Werkzeugschafts größer als der Schaftdurchmesser des zweiten Werkzeugschafts ist. Vorzugsweise entspricht der große Schaftdurchmesser annähernd einem Durchmesser des Schraubenkopfs, während der kleine Schaftdurchmesser vorzugsweise annähernd einem Durchmesser eines Schraubenschafts oder einem Kerndurchmesser der Schraube entspricht oder kleiner als letzterer ist.

Der erste Werkzeugschaft hat einen endseitigen Schrauben-Eingriffsabschnitt/Schraubenkopf-Eingriffsabschnitt, welcher zur Übertragung eines Drehmoments auf eine Schraube, insbesondere einen Schraubenkopf der Schraube, vorgesehen und ausgebildet ist. Vorzugsweise kann der endseitige Schrauben-Eingriffsabschnitt/Schraubenkopf-Eingriffsabschnitt des ersten Werkzeugschafts drehbar sowie dazu vorgesehen und ausgebildet sein, eine Druckkraft axial auf die Schraube, insbesondere den Schraubenkopf zu übertragen. Dadurch kann das bei Drehung des ersten Werkzeugschafts aus der Druckkraft entstehende Reibmoment (zwischen dem ersten Werkzeugschaft und der Schraube) von dem ersten Werkzeugschaft an die Schraube übertragen werden.

Der zweite Werkzeugschaft hat einen endseitigen Schrauben-Eingriffsabschnitt/Schraubenschaft-Eingriffsabschnitt, welcher dazu vorgesehen und ausgebildet ist, eine Druckkraft axial auf die Schraube, insbesondere ein dem Schraubenkopf gegenüberliegendes Schraubenende/Schraubenschaftende der Schraube zu übertragen. Der zweite Werkzeugschaft ist derart axial zum ersten Werkzeugschaft sowie gegenläufig zu diesem ausrichtbar oder ausgerichtet, dass dessen endseitiger Schrauben-Eingriffsabschnitt dem endseitigen Schrauben-Eingriffsabschnitt des ersten Werkzeugschafts axial gegenüberliegt und dazu vorgesehen und ausgebildet ist, die Druckkraft axial in Richtung hin zum ersten Werkzeugschaft auf die Schraube zu übertragen.

Mit anderen Worten haben der etwa stempelartige erste Werkzeugschaft und der etwa stempelartige zweite Werkzeugschaft eine gemeinsame Längsachse und sind voneinander axial so beabstandet, dass der Schrauben-Eingriffsabschnitt des ersten Werkzeugschafts dem Schrauben-Eingriffsabschnitt des zweiten Werkzeugschafts zugewandt ist. Somit kann die Schraube längsausgerichtet zwischen dem ersten Werkzeugschaft und dem zweiten Werkzeugschaft angeordnet werden. Darüber hinaus ist der zweite Werkzeugschaft entlang seiner Längsachse in Richtung zu dem ersten Werkzeugschaft hin verlagerbar, so dass die axial in Richtung zu dem ersten Werkzeugschaft hinwirkende Druckkraft auf die Schraube, und damit von der Schraube auf den ersten Werkzeugschaft, übertragbar ist. Folglich entspricht eine zwischen dem Schrauben-Eingriffsabschnitt des ersten Werkzeugschafts und der Schraube wirkende Kraft einer Summe der durch den ersten Werkzeugschaft und den zweiten Werkzeugschaft jeweils in Richtung zu dem gegenüberliegenden/gegenläufigen Werkzeugschaft gerichteten aufbringbaren Druckkraft.

Der Kern der Offenbarung besteht demzufolge darin, dass das Schraubwerkzeug derart vorgesehen und ausgebildet ist, dass die Schraube axial zwischen dem ersten Werkzeugschaft und dem zweiten Werkzeugschaft eingespannt werden kann. Dies hat den Vorteil, dass der zwischen dem Schraubwerkzeug und der Schraube wirkende Axialdruck unter Umgehung des Innengewindes (und Vermeidung eines dadurch erhöhten Gewindereibmoments) vergrößert werden kann. Einfach gesagt wird die Normalkraft auf die Oberfläche der Schraube, insbesondere des Schraubenkopfs erhöht, während die auf die Schraube (als Ganzes) in Richtung zu dem zweiten Werkzeugschaft hinwirkende Axialkraft gleich bleibt bzw. durch die Druckkraft des zweiten Werkzeugschafts ausgeglichen wird.

In noch andere Worten ausgedrückt hat das offenbarungsgemäße Schraubwerkzeug zwei axial zueinander (auf einer gemeinsamen Achse) sich erstreckender, separater Werkzeugschäfte (co-axial), ein gemeinsames Werkzeugschaft-Lagerungsgestell, an welchem die Werkzeugschäfte (bevorzugt drehbar) gehalten und in Axialrichtung aufeinander zu bewegbar sind, um zwischen sich eine Schraube axial einzuspannen (geschlossener Kraftfluss über das Werkzeugschaft-Lagerungsgestell, die beiden Werkzeugschäfte und die dazwischen eingespannte Schraube). Die über das Werkzeugschaft-Lagerungsgestell auf die Werkzeugschäfte axial (mittels einer Druckkraftvorrichtung des Werkzeugschaft-Lagerungsgestells) aufbringbare Einspannkraft ist bevorzugt so (manuell oder automatisch) einstellbar (z.B. über eine Vorspannfeder, Hydraulik-/Pneumatikstempel oder eine Spannschraube), dass zwischen zumindest dem einen, schraubenkopfseitigen Werkzeugschaft (bzw. dessen Schraub-Eingriffsabschnitt) und der Schraube eine Reibkraft erzeugt wird, die die Übertragung eines bestimmten Einschraub-Drehmoments (von dem schraubenkopfseitigen Werkzeugschaft oder beiden Werkzeugschäften) auf die Schraube ermöglicht.

Dies hat den Vorteil, dass die Schraube allein oder zumindest überwiegend durch reibschlüssige Kraftübertragung mit dem anmeldungsgemäßen Schraubwerkzeug eingeschraubt werden kann, wodurch Freiheiten hinsichtlich der Gestaltung der Schraube, insbesondere einer Form eines Schraubenkopfes entstehen. So lässt sich insbesondere der Schraubenkopf vorzugsweise mit ebener/unprofilierter Oberfläche ausbilden, so dass zwischen dem Schraubenkopf und dem (ebenfalls vorzugsweise unprofilierten/keinen Formschluss erzeugenden) Schraub-Eingriffsabschnitt des schraubenkopfseitigen Werkzeugschafts im Wesentlichen nur Reibkräfte wirken können, wodurch insbesondere Nachteile vermieden werden, die aufgrund eines zur formschlüssigen Kraftübertragung notwendigen, oftmals kantigen Außen- oder Innenprofils des Schraubenkopfes entstehen. Vorzugsweise kann der Schraubenkopf im Wesentlichen rotationssymmetrisch ausgebildet werden, wodurch eine Bildung von Spalten, Schlitzen, Taschen oder ähnlichem vermieden wird, da sich aus diesen Gewebereste, Schmutz und Körperflüssigkeiten bei einer Wiederaufbereitung des Instruments nicht zuverlässig und vollständig entfernen lassen.

Gemäß einer bevorzugten Ausführungsform kann der Schrauben-Eingriffsabschnitt des ersten Werkzeugschafts eine im Wesentlichen konkav gekrümmte, insbesondere sphärische (glatte/unprofilierte) oder kegelstumpfförmige Oberfläche haben. Der Schrauben-Eingriffsabschnitt des ersten Werkzeugschafts kann vorzugsweise derart vorgesehen und ausgebildet sein, dass die Form des Schrauben-Eingriffsabschnitts der Form des Schraubenkopfes im Wesentlichen entspricht. Das heißt also, dass der Schraubenkopf vorzugsweise eine im Wesentlichen konvex gekrümmte, insbesondere sphärische (unprofilierte) Oberfläche hat. Alternativ oder zusätzlich kann der Schrauben-Eingriffsabschnitt des zweiten Werkzeugschafts gemäß einer bevorzugten Ausführungsform eine im Wesentlichen ebene oder konkav gekrümmte, insbesondere sphärische (unprofilierte) Oberfläche haben. Der Schrauben-Eingriffsabschnitt des zweiten Werkzeugschafts kann vorzugsweise derart vorgesehen und ausgebildet sein, dass die Form des Schrauben-Eingriffsabschnitt der Form des Schraubenendes entspricht. Das heißt also, dass das Schraubenende vorzugsweise eine im Wesentlichen ebene oder konvex gekrümmte, insbesondere sphärische Oberfläche hat. Dadurch können/kann der erste Werkzeugschaft und/oder der zweite Werkzeugschaft in vorteilhafter Weise geführt und zentriert ausgerichtet werden. Es erfolgt also bevorzugt eine Selbstzentrierung bei der Montage bzw. beim Einspannvorgang der Schraube zwischen den axial beabstandeten Werkzeugschäften. Ein weiterer Vorteil der weichkantigen/gekrümmten Oberflächenausbildung des ersten Werkzeugschafts und/oder zweiten Werkzeugschafts liegt darin, dass Beschädigungen beim Abrutschen des Schraubwerkzeugs und somit eine eventuelle Nachbearbeitung der Schraube entfallen kann, die zur Gewährleistung einer für medizinische, insbesondere chirurgische Instrumente/Geräte geforderte Oberflächenqualität erforderlich ist.

Gemäß einer bevorzugten Ausführungsform betrifft die vorliegende Offenbarung das Schraubwerkzeug mit einer Schraube, die dazu vorgesehen und ausgebildet ist, von dem Schrauben-Eingriffsabschnitt des ersten Werkzeugschafts das Drehmoment und die Druckkraft von dem Schrauben-Eingriffsabschnitt des zweiten Werkzeugschafts aufzunehmen. Insbesondere kann die Schraube einen im Wesentlichen sphärischen (oder kugelsegmentförmigen) Schraubenkopf haben.

Gemäß einer bevorzugten Ausführungsform kann das Schraubwerkzeug eine Anziehvorrichtung (Abschnitt des Werkzeugschaft-Lagerungsgestells/ Werkzeugschaft-Haltevorrichtung zum Einspannen einer Schraube zwischen den axial beabstandeten Werkzeugschäften) haben. Die Anziehvorrichtung kann vorzugsweise vorgesehen und ausgebildet sein, um einen Drehwinkel und/oder das Drehmoment des Schrauben-Eingriffsabschnitts des ersten Werkzeugschafts einzustellen. Alternativ oder zusätzlich kann die Anziehvorrichtung vorzugsweise vorgesehen und ausgebildet sein, um einen Drehwinkel und/oder ein Drehmoment des Schrauben-Eingriffsabschnitt des zweiten Werkzeugschafts zu messen. Somit kann eingestellt werden, wie weit und/oder wie fest die Schraube (in ein medizinisches Instrument) eingeschraubt wird, d.h. welcher Gang der Schraube im Gewindeeingriff ist und/oder wie groß ein durch die Schraubverbindung entstehendes Widerstandsmoment, insbesondere zweier dadurch beweglich/schwenkbar miteinander verbundener Bauteile eines chirurgischen Instruments, ist. D.h., die Anziehvorrichtung kann nach Art einer Durchrutschkupplung verwendet werden, um ein Überdrehen der einzudrehenden Schraube zu vermeiden.

Gemäß einer bevorzugten Ausführungsform kann das Schraubwerkzeug eine (vorstehend bereits angedeutete) Druckkraftvorrichtung (weiterer Abschnitt des Werkzeugschaft-Lagerungsgestells/Werkzeugschaft-Haltevorrichtung) haben. Die Druckkraftvorrichtung ist vorzugsweise vorgesehen und ausgebildet sein, um die Druckkraft (von dem zweiten Werkzeugschaft axial in Richtung hin zum ersten Werkzeugschaft auf die Schraube und/oder von dem ersten Werkzeugschaft axial in Richtung hin zum zweiten Werkzeugschaft auf die Schraube) einzustellen, zu berechnen und/oder zu messen. Mit anderen Worten ist die Druckkraftvorrichtung vorzugsweise vorgesehen und ausgebildet, eine auf die Schraube wirkende Axialspannkraft des Schraubwerkzeugs einzustellen, zu berechnen und/oder zu messen. Insbesondere kann die Druckkraftvorrichtung derart ausgebildet sein, dass ein Axialabstand zwischen dem ersten Werkzeugschaft und dem zweiten Werkzeugschaft, beispielsweise mittels eines Gewindetriebs, etwa entgegen der Rückstellkraft einer Feder, veränderbar/einstellbar ist. So kann die Axialspannkraft/axiale Druckkraft in Abhängigkeit der Länge der in dem Schraubwerkzeug einzuspannenden Schraube festgelegt werden. Die Druckkraftvorrichtung kann manuell oder vorzugsweise automatisiert betätigbar sein.

Gemäß einer bevorzugten Ausführungsform können der erste Werkzeugschaft und der zweite Werkzeugschaft axial zueinander beweglich, vorzugsweise axialverschieblich oder schwenkbar, gelagert sein. Somit können die Schraube-Eingriffsabschnitte in einfacher Weise, etwa nach Art eine Presse oder nach Art einer Zange, aufeinander zu und voneinander wegbewegt werden.

Gemäß einer bevorzugten Ausführungsform kann der Schrauben-Eingriffsabschnitt des ersten Werkzeugschafts in Axialrichtung zu dem zweiten Werkzeugschaft hin federvorgespannt sein. Alternativ oder zusätzlich kann der Schrauben-Eingriffsabschnitt des zweiten Werkzeugschafts gemäß einer bevorzugten Ausführungsform in Axialrichtung zu dem ersten Werkzeugschaft hin federvorgespannt sein. Dies hat den Vorteil, dass durch die Federvorspannung eine von dem Schrauben-Eingriffsabschnitt übertragbare definierte Druckkraft, und somit auch ein definiertes Anzugsmoment, eingestellt werden kann. Darüber hinaus können Beschädigungen an der Schraube durch eine zu hohe Druckkraft vermieden werden.

Gemäß einer bevorzugten Ausführungsform kann der erste Werkzeugschaft mit dem zweiten Werkzeugschaft integral um seine Längsachse drehbar verbunden sein. Das heißt, dass der zweite Werkzeugschaft bei Drehung des ersten Werkzeugschafts mitdreht. So kann auch von dem Schrauben-Eingriffsabschnitt des zweiten Werkzeugschafts ein Drehmoment zum Einschrauben der Schraube auf die Schraube übertragen werden.

Gemäß einer alternativen bevorzugten Ausführungsform kann der erste Werkzeugschaft relativ zu dem zweiten Werkzeugschaft um seine Längsachse relativ drehbar sein. Das heißt, dass der zweite Werkzeugschaft bei Drehung des ersten Werkzeugschafts stillsteht/nicht mitdreht. Somit dient der zweite Werkzeugschaft nur als Gegenhalter zum Aufbringen der axialen Druckkraft. Alternativ kann der zweite Werkzeugschaft um seine Längsachse unabhängig von dem ersten Werkzeugschaft um seine Längsachse drehbar sein. So kann auch über den zweiten Werkzeugschaft ein Drehmoment auf die Schraube aufgebracht werden.

Die Aufgabe der vorliegenden Offenbarung wird auch durch ein Montageverfahren zum Einschrauben einer Schraube in ein Innengewinde eines medizinischen, zwei Branchen aufweisenden Instruments, mit einem, vorzugsweise dem beschriebenen Schraubwerkzeug gelöst, um die zwei Branchen durch die Schraube so miteinander zu verbinden, dass die zwei Branchen zueinander um die Schraube als Scharnier verschwenkbar sind. Das anmeldungsgemäße Montageverfahren weist die folgenden Schritte auf:
- axiales Aufeinander-Zubewegen eines ersten Werkzeugschafts und eines zweiten Werkzeugschafts des Schraubwerkzeugs zum Einspannen der Schraube zwischen ihrem Schraubenkopf und ihrem Schraubenschaft in das Schraubwerkzeug, so dass eine Druckkraft axial von dem zweiten Werkzeugschaft in Richtung hin zum ersten Werkzeugschaft auf die Schraube übertragen wird,
- Drehen des Schraubwerkzeugs, insbesondere des ersten und ggf. zweiten Werkzeugschafts, relativ zu dem Instrument um eine Schraubenlängsachse, um ein Drehmoment (insbesondere zur Einstellung eines Anzugsmoments oder einer Einschraubtiefe der Schraube) auf die in das Schraubwerkzeug eingespannte Schraube zu übertragen und die Schraube in das Innengewinde einzuschrauben, und
- axiales Auseinanderbewegen des ersten Werkzeugschafts und des zweiten Werkzeugschafts zum Ausspannen der Schraube aus dem Schraubwerkzeug.

Zusätzlich weist eine erste Branche der beiden Branchen ein erstes Durchgangsloch auf und eine zweite Branche der beiden Branchen ein zweites Durchgangsloch auf, an dem das Innengewinde ausgebildet ist. Gemäß einer bevorzugten Ausführungsform des Montageverfahrens weist es den folgenden Schritt auf:
- axiales Bewegen des zweiten Werkzeugschafts auf den ersten Werkzeugschaft zu und durch das zweite Durchgangsloch hindurch, bevor die Schraube in das Schraubwerkzeug eingespannt sowie in das Innengewinde eingesetzt wird, oder
- Hindurchführen der Schraube durch das erste Durchgangsloch hindurch und Einsetzen der Schraube in das Innengewinde, bevor der zweite Werkzeugschaft durch das zweite Durchgangsloch hindurch auf den ersten Werkzeugschaft axial zubewegt wird und die Schraube in das Schraubwerkzeug eingespannt wird.

Demnach kann die Schraube zuerst in das Schraubwerkzeug gespannt werden, bevor sie in das Innengewinde eingesetzt und eingeschraubt wird, oder die Schraube kann zuerst in das Innengewinde eingesetzt werden, bevor sie in das Schraubwerkzeug eingespannt und eingeschraubt wird.

Vorzugsweise kann die Schraube in ihrer eingeschraubten Position gesichert werden, beispielsweise durch Laserschweißen und/oder durch Klebstoff, der vor dem Einspannen in das Schraubwerkzeug aufgebracht werden kann.

Zusammenfassend kann dazu ergänzt werden, dass ein reinigungsoptimiertes Verbindungselement für chirurgische Instrumente bereitgestellt wird, bei dem das Problem gelöst wird, wonach sich in einem Schlitz der Schraube Gewebereste, Schmutz und Körperflüssigkeiten sammeln und anhaften können, die beim Aufbereitungsprozess nicht vollständig entfernt werden. Nachteile von bisherigen Verbindungselementen liegen unter anderem darin, dass sich der scharfkantig ausgeführte Schlitzgrund und Schlitze nicht zuverlässig reinigen lassen, dass in der Massenproduktion nicht vermeidbare Montagefehler, wie das Abrutschen eines Schraubendrehers, durch manuell, etwa durch Polieren, nachbearbeitet werden müssen. Somit stellt sich die Aufgabe, eine Kombination aus einem Design eines Verbindungselementkopfs und einem geeigneten Montageverfahren zu ermöglichen, bei dem keine tiefen und am Grund scharfkantigen Taschen, Schlitze oder dergleichen vorhanden sind und die aufgebrachten Anzugsmomente/Montagekräfte fein einstellbar/dosierbar sind. Dabei können der Schraubenkopf und das Gewindeende sphärisch gestalten sein, so dass die sphärische Auflage das System führt und sich von selbst zentriert, wenn die Schraube beim Montageprozess zwischen ihrem Kopf und dem Gewindeende gespannt wird. Die Kraftübertragung erfolgt über die über ein Werkzeug aufgebracht Reibungskraft. Dabei kann das Anzugsmoment oder der Gang bzw. das Widerstandsmoment der beweglichen Teile feinfühlig eingestellt und/oder über eine Sensorik gemessen und/oder protokoliert werden. Die Schraubverbindung kann in dieser Schraubenstellung, falls erforderlich, mittels Klebstoff, einem Laserschweißpunkt oder dergleichen gesichert werden. Da der Schraubenkopf ohne einen Schlitz oder ein ähnliches Element der Kraftübertragung (Formschlussmittel) ausgeführt ist muss das Verbindungselement nicht nachbearbeitet werden, um die für chirurgische Instrumente/Geräte geforderte Oberflächenqualität zu erreichen. Somit ergibt sich eine prozesssichere/reproduzierbare maschinelle Herstellbarkeit, eine Vereinfachung der Montage durch Erhöhung des Mechanisierungsgrads bei der Herstellung sowie bessere Reinigungseigenschaften.

### Kurzbeschreibung der Figuren

Fign. 1 bis 4 zeigen perspektivische Darstellungen eines Ausschnitts eines anmeldungsgemäßen Schraubwerkzeugs zu verschiedenen Zeitpunkten eines damit ausgeführten Montageverfahrens,
Fign. 5 bis 7 zeigen weitere perspektivische Darstellungen des Schraubwerkzeugs zu verschiedenen Zeitpunkten des damit ausgeführten Montageverfahrens,
Fign. 8 und 9 zeigen perspektivische Darstellungen einer mit dem Schraubwerkzeug einschraubbaren Schraube und eines Instruments, in das die Schraube eingeschraubt ist, und
Fign. 10 bis 12 zeigen perspektivische Darstellungen des Schraubwerkzeugs mit zwei axial sich erstreckenden Werkzeugschäften und einem (schraubzwingenartigen) Werkzeugschaft-Lagerungs-/Haltegestell.

### Beschreibung von bevorzugten Ausführungsformen

Nachstehend werden bevorzugte Ausführungsformen der vorliegenden Offenbarung auf der Basis der zugehörigen Figuren beschrieben.

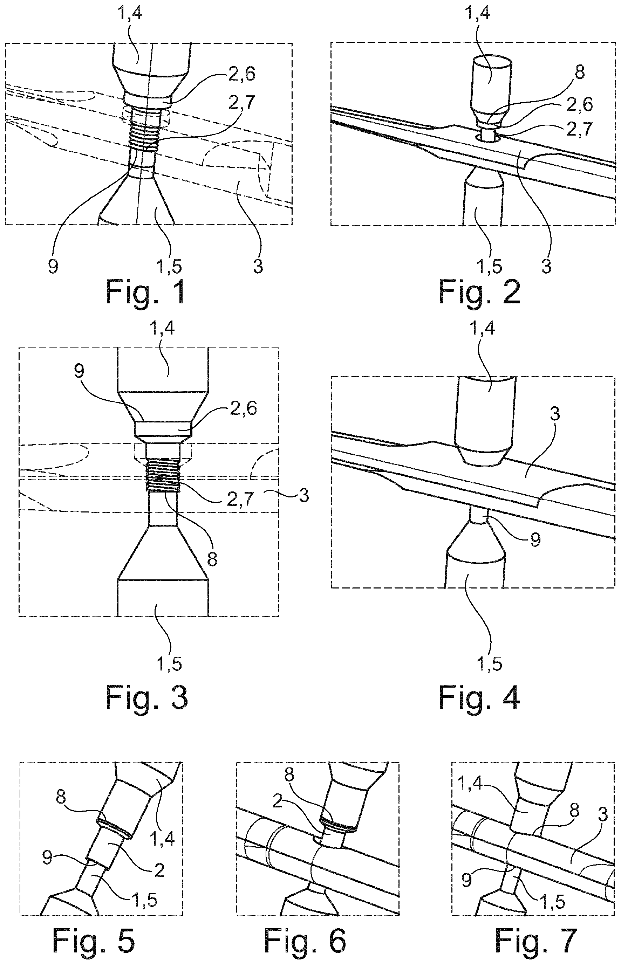

Fign. 1 bis 7 zeigen perspektivische Darstellungen eines Ausschnitts eines anmeldungsgemäßen Schraubwerkzeugs 1 zu verschiedenen Zeitpunkten eines damit ausgeführten Montageverfahrens. Das Schraubwerkzeug 1 dient zum Einschrauben einer ein Außengewinde aufweisenden Schraube 2 in ein Innengewinde. Das Innengewinde ist in den dargestellten Ausführungsformen an einem chirurgischen Instrument 3 ausgebildet. In den dargestellten Ausführungsformen legt die Schraube 2 zwei Bestandteile des Instruments 3 hier zueinander beweglich, insbesondere verschwenkbar fest.

Das Schraubwerkzeug 1 hat einem ersten Werkzeugschaft 4, der einen großen Schaftdurchmesser aufweist, und einem zweiten Werkzeugschaft 5, der einen kleinen Schaftdurchmesser aufweist. Der Schaftdurchmesser des ersten Werkzeugschafts 4 ist demnach größer als der Schaftdurchmesser des zweiten Werkzeugschafts 5. Vorzugsweise entspricht der große Schaftdurchmesser annähernd einem Durchmesser eines Schraubenkopfs 6 der Schraube 2, während der kleine Schaftdurchmesser vorzugsweise annähernd einem Durchmesser eines Schraubenschafts 7 oder einem Kerndurchmesser der Schraube 2 entspricht (oder geringfügig kleiner ist).

Der erste Werkzeugschaft 4 hat einen endseitigen Schrauben-Eingriffsabschnitt 8 hat, der zur Übertragung eines Drehmoments auf die Schraube 2, insbesondere den Schraubenkopf 7, vorgesehen und ausgebildet ist. Der Schrauben-Eingriffsabschnitt 8 kann vorzugsweise einer Form des Schraubenkopfes 7 entsprechen und insbesondere eine konkav gekrümmte Oberfläche besitzen. Die konkav gekrümmte Oberfläche kann vorzugsweise sphärisch sein, auch wenn dies in den Figuren nicht explizit dargestellt ist. Der zweite Werkzeugschaft 5 ist axial zum ersten Werkzeugschaft 4 sowie gegenläufig zu diesem ausgerichtet. Der erste Werkzeugschaft 4 und der zweite Werkzeugschaft 5 haben demnach eine gemeinsame Längsachse und sind axial zueinander beabstandet. Der zweite Werkzeugschaft 5 ist derart ausgerichtet, dass dessen endseitiger Schrauben-Eingriffsabschnitt 9 dem endseitigen Schrauben-Eingriffsabschnitt 8 des ersten Werkzeugschafts 4 axial gegenüberliegt und dazu vorgesehen und ausgebildet ist, eine Druckkraft axial in Richtung hin zum ersten Werkzeugschaft 4 auf die Schraube 2 zu übertragen. Das heißt, dass die Schraube 2 axial zwischen dem ersten Werkzeugschaft 4 und dem zweiten Werkzeugschaft 5 angeordnet werden kann oder angeordnet ist. Die Schraube 2 kann axial zwischen dem ersten Werkzeugschaft 4 und dem zweiten Werkzeugschaft 5 eingespannt werden. Im eingespannten Zustand (vgl. Fign. 1 bis 4) liegt der Schraubenkopf 6 an dem Schrauben-Eingriffsabschnitt 8 und der Schraubenschaft/ein Schraubenende 7 an dem Schrauben-Eingriffsabschnitt 9 des Schraubwerkzeugs 1 an.

Der erste Werkzeugschaft 4 und der zweite Werkzeugschaft 5 können axial zueinander beweglich, vorzugsweise axialverschieblich oder schwenkbar, gelagert sein. Beispielsweise können der erste Werkzeugschaft 4 und der zweite Werkzeugschaft 5 nach Art einer Presse oder nach Art einer Zange ausgebildet sein.

Vorzugsweise kann der Schrauben-Eingriffsabschnitt 8 des ersten Werkzeugschafts 4 in Axialrichtung zu dem zweiten Werkzeugschaft 5 hin federvorgespannt sein, auch wenn dies nicht dargestellt ist. Vorzugsweise kann der Schrauben-Eingriffsabschnitt 9 des zweiten Werkzeugschafts 5 in Axialrichtung zu dem ersten Werkzeugschaft 4 hin federvorgespannt sein, auch wenn dies nicht dargestellt ist. Beispielsweise kann der erste Werkzeugschaft mit dem zweiten Werkzeugschaft integral um seine Längsachse drehbar, d.h. drehgekoppelt, verbunden sein. Alternativ kann der erste Werkzeugschaft 4 relativ zu dem zweiten Werkzeugschaft 5 um seine Längsachse relativ drehbar sein.

Fig. 5 zeigt das beschriebene Schneidwerkzeug 1, in das die Schraube 2 zwischen die beiden Werkzeugschäfte 4, 5 eingespannt ist, ohne Darstellung des Instruments 3, in das die Schraube 2 eingeschraubt ist/werden soll. Fig. 6 entspricht im Wesentlichen Fig. 2, in der ein Zustand während dem Einschrauben der Schraube 2 dargestellt ist, während Fig. 7 im Wesentlichen Fig. 4 entspricht, in der ein Zustand mit eingeschraubter Schraube 2 dargestellt ist.

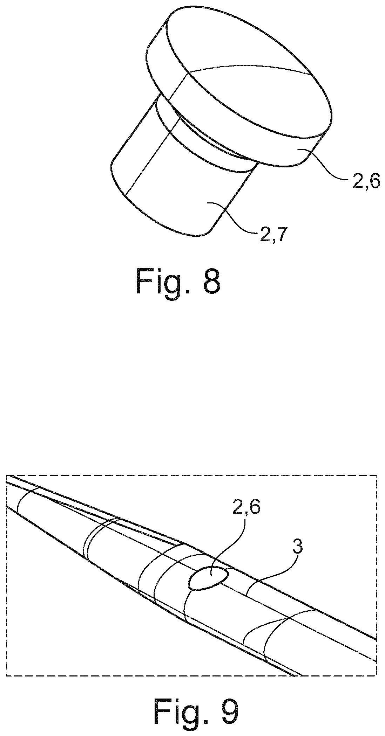

Fig. 8 zeigt eine perspektivische Darstellung der Schraube 2 in einer ersten bevorzugten Ausführungsform. Der Schraubenkopf 6 der Schraube 2 bzw. dessen Oberfläche ist konvex gekrümmt. Insbesondere kann der Schraubenkopf 6 im Wesentlichen rotationssymmetrisch, beispielsweise sphärisch ausgebildet sein. Der Schraubenkopf 6 weist in dieser Ausführungsform keine Formschlussangriffsflächen auf.

Fig. 9 zeigt eine perspektivische Darstellung des Instruments 3 mit der darin eingeschraubten Schraube 2 in der ersten bevorzugten Ausführungsform. Durch die konvexe Krümmung und die kreisförmige Ausbildung des Schraubenkopfes 6 entstehen keine Spalte oder Taschen, in denen sich Schmutz, Gewebereste oder Flüssigkeiten sammeln könnte.

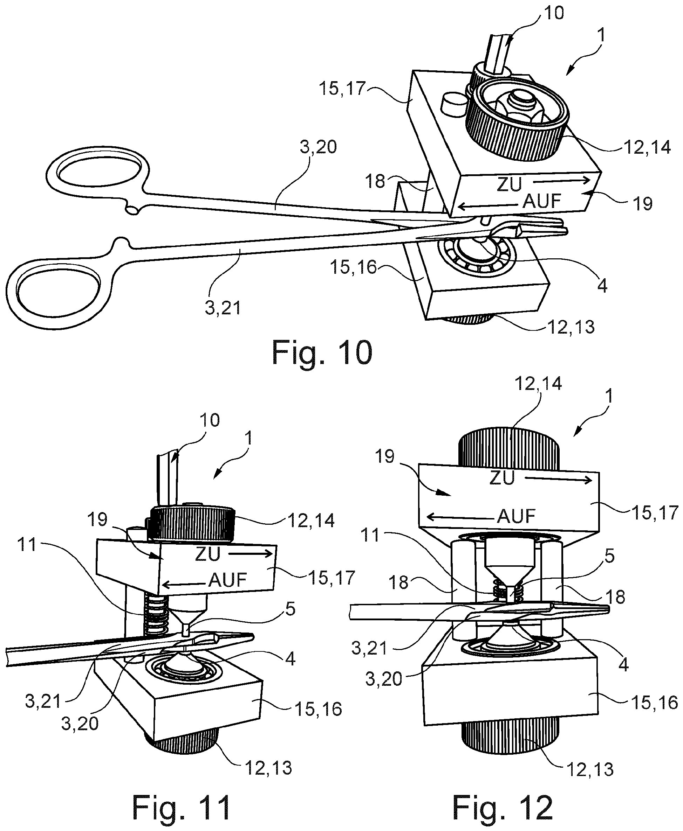

Fig. 10 bis 12 zeigen perspektivische Darstellungen des Schraubwerkzeugs 1. Das Schraubwerkzeug 1 kann zusätzlich eine Druckkraftvorrichtung 10 aufweisen. Die Druckkraftvorrichtung 10 ist dazu vorgesehen und ausgebildet, die Druckkraft (von dem zweiten Werkzeugschaft 5 axial in Richtung hin zum ersten Werkzeugschaft 4 auf die Schraube 2 bzw. von dem ersten Werkzeugschaft 4 axial in Richtung hin zum zweiten Werkzeugschaft 5 auf die Schraube 2) einzustellen, zu berechnen und/oder zu messen. Mit anderen Worten ist die Druckkraftvorrichtung 10 dazu vorgesehen und ausgebildet, eine auf die Schraube 2 wirkende Axialspannkraft des Schraubwerkzeugs 1 einzustellen, zu berechnen und/oder zu messen. Insbesondere ist die Druckkraftvorrichtung 10 derart ausgebildet, dass ein Axialabstand zwischen dem ersten Werkzeugschaft 4 und dem zweiten Werkzeugschaft 5, beispielsweise mittels eines Gewindetriebs, veränderbar/einstellbar ist. So kann die Axialspannkraft/axiale Druckkraft in Abhängigkeit der Länge der in dem Schraubwerkzeug 1 einzuspannenden Schraube 2 festgelegt werden. Vorzugsweise kann die Druckkraftvorrichtung 10 eine Spannfeder 11 aufweisen, entgegen deren Rückstellkraft der Axialabstand zwischen dem ersten Werkzeugschaft 4 und dem zweiten Werkzeugschaft 5 verändert, in der dargestellten Ausführungsform verringert, werden kann. Die Druckkraftvorrichtung 10 kann manuell oder vorzugsweise automatisiert betätigbar sein.

Das Schraubwerkzeug 1 kann zusätzlich eine Anziehvorrichtung 12 aufweisen. Die Anziehvorrichtung 12 ist dazu vorgesehen und ausgebildet ist, einen Drehwinkel und/oder das Drehmoment des Schrauben-Eingriffsabschnitts 8 des ersten Werkzeugschafts 4 und/oder des Schrauben-Eingriffsabschnitts 9 des zweiten Werkzeugschafts 5 einzustellen und/oder zu messen. Die Anziehvorrichtung 12 kann manuell oder vorzugsweise automatisiert betätigbar sein. Zur Einstellung einer definierten Einschraubtiefe der Schraube 2, was auch als Einstellung eines Gangs bezeichnet wird, und/oder zur Einstellung eines definierten Anzugsmoment, mit dem die Schraube 2 eingeschraubt wird, kann das durch das Schraubwerkzeug 1 auf die Schraube 2 übertragende Drehmoment, etwa nach Art eines Drehmomentschlüssels, und/oder den durch das Schraubwerkzeug 1 auf die Schraube 2 übertragenden Drehwinkel, etwa nach Art eines Drehwinkelmessers, mit der Anziehvorrichtung 12 eingestellt und gemessen werden. Somit kann eingestellt werden, wie weit die Schraube 2 eingeschraubt wird, d.h. welcher Gang der Schraube 2 im Gewindeeingriff ist.

In der dargestellten Ausführungsform weist die Anziehvorrichtung 12 ein erstes Anziehrad 13 auf, das drehfest mit dem ersten Werkzeugschaft 4 verbunden ist. Ist die Schraube 2 in das Schraubwerkzeug 1 eingespannt und wird das erste Anziehrad 13 relativ zu dem Instrument 3 gedreht, wird die Schraube 2 mit dem aus der axialen Druckkraft resultierenden Reibmoment in das Instrument 3 eingeschraubt. In der dargestellten Ausführungsform weist die Anziehvorrichtung 12 ein zweites Anziehrad 14 auf, das drehfest mit dem zweiten Werkzeugschaft 5 verbunden ist. Ist die Schraube 2 in das Schraubwerkzeug 1 eingespannt und wird das zweite Anziehrad 14 relativ zu dem Instrument 3 gedreht, wird die Schraube 2 mit dem aus der axialen Druckkraft resultierenden Reibmoment in das Instrument 3 eingeschraubt. Durch Drehung der beiden Anziehräder 13, 14 kann das maximal übertragbare Reibmoment erhöht werden. Die beiden Anziehräder 13, 14 können integral miteinander drehbar/antreibbar sein oder separat/individuell voneinander drehbar/antreibbar sein. Alternativ kann auch nur ein Anziehrad vorgesehen sein, so dass der erste Werkzeugschaft 4 bei Drehung relativ zu dem Instrument auch relativ zu dem zweiten Werkzeugschaft 5 verdreht wird (und der zweite Werkzeugschaft 5 nur als Gegenhalter zum Aufbringen der axialen Druckkraft dient).

Das Schraubwerkzeug 1 kann eine Halterung 15 aufweisen, an welcher der erste Werkzeugschaft 4 und/oder der zweite Werkzeugschaft 5 angebracht sind. In der dargestellten Ausführungsform weist die Halterung 15 eine erste Aufnahme 16, an der der erste Werkzeugschaft 4 drehbar gelagert ist sowie eine zweite Aufnahme 17 auf, an der der zweite Werkzeugschaft 5 drehbar gelagert ist. Ein Axialabstand der beiden Aufnahmen 16, 17 ist über die Druckkraftvorrichtung 10 einstellbar. Die Halterung 15 weist eine Axialführung 18 auf, hier in Form von zwei parallel zu der Drehachse ausgerichteten Stangen, durch welche eine Einstellung des Axialabstands parallel zu der Drehachse geführt vorgegeben ist. An der Halterung 15 ist eine Skala 19 ausgebildet, die eine Ein- bzw. Ausdrehrichtung (close bzw. open) des Schraubwerkzeugs 1 indiziert.

Wie aus Fig. 10 ersichtlich, dient das anmeldungsgemäße Schraubwerkzeug 1 zum Einschrauben der Schraube 2 in das Innengewinde des Instruments 3, welches zwei Branchen 20, 21 besitzt, um die zwei Branchen 20, 21 des Instruments 3 so miteinander zu verbinden, dass die zwei Branchen 18, 19 zueinander verschwenkbar sind. Dabei kann durch die Einstelltiefe der Schraube 2 bzw. den Gang der Schraube 2 eingestellt werden, wie groß ein durch die Schraubverbindung entstehendes Widerstandsmoment der beiden dadurch beweglich/schwenkbar miteinander verbundenen Branchen 20, 21 des Instruments 3 ist. Vorzugsweise weist die erste Branche 20 der beiden Branchen 20, 21 ein erstes Durchgangsloch auf. Vorzugsweise weist die zweite Branche 21 der beiden Branchen 20, 21 ein zweites Durchgangsloch auf, an dem das Innengewinde ausgebildet ist. Insbesondere ist das erste Durchgangsloch kleiner als ein Durchmesser des Schraubenkopfes 6, so dass die beiden Branchen 20, 21 verschwenkbar zueinander festgelegt werden können, indem der Schraubenkopf 6 axial an dem ersten Durchgangsloch anliegt, und der Schraubenschaft 7 das erste Durchgangsloch durchgreift sowie in das Innengewinde in dem zweiten Durchgangsloch eingreift.

Die vorliegende Offenbarung betrifft auch ein Montageverfahren zum Einschrauben der Schraube 2 in das Innengewinde des die zwei Branchen 20, 21 aufweisenden Instruments 3, um die zwei Branchen 20, 21 des Instruments 3 so miteinander zu verbinden, dass sie zueinander verschwenkbar sind. In einem Schritt des Montageverfahrens werden der erste Werkzeugschaft 4 und der zweite Werkzeugschaft 5 axial aufeinander zubewegt, um die Schraube 2 zwischen ihrem Schraubenkopf 6 und ihrem Schraubenschaft 7 in das Schraubwerkzeug 1 so einzuspannen, dass eine Druckkraft axial von dem zweiten Werkzeugschaft 5 in Richtung hin zum ersten Werkzeugschaft 4 auf die Schraube 2 (und von dem ersten Werkzeugschaft 4 in Richtung hin zum zweiten Werkzeugschaft auf die Schraube 2) übertragen wird. Der Schrauben-Eingriffsabschnitt 8 des ersten Werkzeugschafts 4 wird vorzugsweise so bewegt, dass er über eine gesamte Schraubenkopfoberfläche an diesem anliegt. Der Schrauben-Eingriffsabschnitt 9 des zweiten Werkzeugschafts 5 wird vorzugsweise so bewegt, dass er über eine gesamte (axiale) Oberfläche des Schraubenschafts 7 an diesem anliegt.

In einem nachgelagerten Schritt des Montageverfahrens wird das Schraubwerkzeug 1, insbesondere der erste Werkzeugschaft 4 sowie vorzugsweise der zweite Werkzeugschaft 5, relativ zu dem Instrument 3 um eine Schraubenlängsachse der Schraube 2 gedreht, um ein Drehmoment, insbesondere zur Einstellung eines Anzugsmoments oder einer Einschraubtiefe der Schraube 2, auf die in das Schraubwerkzeug 1 eingespannte Schraube 2 zu übertragen und die Schraube 2 in das Innengewinde einzuschrauben. Ist die Schraube 2 zwischen den beiden Schrauben-Eingriffsabschnitten 8, 9 eingespannt, kann das Drehmoment über den Schrauben-Eingriffsabschnitt 8 des ersten Werkzeugschafts 4 auf die Schraube 2 reibschlüssig übertragen werden, indem der Schrauben-Eingriffsabschnitt 8 des ersten Werkzeugschafts 4 zur Erzeugung eines Reibmoments um die Schraubenlängsachse gedreht wird. Der Schrauben-Eingriffsabschnitt 9 des zweiten Werkzeugschafts 5 kann zur Erhöhung des Reibmoments mitdrehen/auch gedreht werden oder zum reinen Aufbringen der axialen (Gegen-)Druckkraft stillstehen (relativ zu dem Instrument 3). Insbesondere wird das Schraubwerkzeug 1 wendelartig bewegt, um die Schraube 2 einzudrehen. Vorzugsweise wird die Schraube 2 so weit in das Instrument 3 eingeschraubt, bis der Rand der Oberfläche des Schraubenkopfs 6 und/oder des Schraubenschafts 7 in Abhängigkeit von der Instrumentenoberfläche, vorzugsweise komplett, aber zumindest teilweise bündig mit einer Oberfläche des Instruments 3 abschließt.

In einem darauffolgenden Schritt des Montageverfahrens werden der erste Werkzeugschaft 4 und der zweite Werkzeugschaft 5 axial auseinanderbewegt, um die Schraube 2 aus dem Schraubwerkzeug 1 auszuspannen.

Vorzugsweise kann der zweite Werkzeugschaft 5 auf den ersten Werkzeugschaft 4 zubewegt und durch das zweite Durchgangsloch (in der das Innengewinde aufweisenden zweiten Branche 21) hindurchgeführt werden, bevor die Schraube 2 in das Schraubwerkzeug 1 eingespannt und in das Innengewinde eingesetzt wird. Der zweite Werkzeugschaft 5 wird demnach zuerst durch das Instrument 3 ("von unten", d.h. einem dem Schraubenkopf 6 gegenüberliegenden Ende des Instruments 3) hindurchgeführt, bevor die Schraube 2 in dem Schraubwerkzeug 1 eingespannt, dann im eingespannten Zustand in das Innengewinde eingesetzt und durch Drehmomentübertragung in das Innengewinde eingeschraubt wird. Insbesondere ist das zweite Durchgangsloch größer als der zweite Werkzeugschaft 5, damit der zweite Werkzeugschaft zum Spannen der Schraube 2 von unten durch das zweite Durchgangsloch geführt werden kann.

Alternativ kann die Schraube zuerst durch das erste Durchgangsloch in der ersten Branche 20 hindurchgeführt und in das Innengewinde in der zweiten Branche 21 eingesetzt werden, bevor der zweite Werkzeugschaft 5 durch das zweite Durchgangsloch hindurch auf den ersten Werkzeugschaft 4 axial zubewegt wird und die Schraube 2 in das Schraubwerkzeug 1 eingespannt wird. Demnach wird die Schraube 2 zuerst "von oben", d.h. von einem dem Schraubenkopf 6 nahen Ende des Instruments 3 in das Innengewinde eingesetzt, bevor der erste Werkzeugschaft 4 und der zweite Werkzeugschaft 5 aufeinander zubewegt werden, um die Schraube 2 einzuspannen. Insbesondere ist das zweite Durchgangsloch größer als der zweite Werkzeugschaft 5, damit der zweite Werkzeugschaft zum Spannen der Schraube 2 von unten durch das zweite Durchgangsloch geführt werden kann. Die Schraube 2 kann vorzugsweise um die Schraubenlängsrichtung gedreht werden, d.h. beispielsweise händisch etwas/um wenige Umdrehungen eingedreht werden, bevor die Schraube 2 eingespannt wird.

Vorzugsweise kann die Schraube 2 in ihrer eingeschraubten Position durch Laserschweißen und/oder durch vor dem Einspannen in das Schraubwerkzeug 1 aufgebrachten Klebstoff gesichert werden. Der Umfang der Erfindung wird durch die Ansprüche definiert.

## Patentansprüche

1. Schraubwerkzeug (1) zum Einschrauben einer Schraube (2), insbesondere in ein Innengewinde eines medizinischen Instruments (3), mit
- einem ersten Werkzeugschaft (4), der vorzugsweise einen großen Schaftdurchmesser aufweist und der einen endseitigen Schrauben-Eingriffsabschnitt (8) hat, der zur Übertragung eines Drehmoments auf die Schraube (2) vorgesehen und ausgebildet ist,
- einem zweiten Werkzeugschaft (5), der vorzugsweise einen kleinen Schaftdurchmesser aufweist und der axial zum ersten Werkzeugschaft (4) sowie gegenläufig zu diesem ausrichtbar oder ausgerichtet ist, derart, dass dessen endseitiger Schrauben-Eingriffsabschnitt (9) dem endseitigen Schrauben-Eingriffsabschnitt (8) des ersten Werkzeugschafts (4) axial gegenüberliegt und dazu vorgesehen und ausgebildet ist, eine Druckkraft axial in Richtung hin zum ersten Werkzeugschaft (4) auf die Schraube (2) zu übertragen, und
- einer Schraube, die dazu vorgesehen und ausgebildet ist, von dem Schrauben-Eingriffsabschnitt (8) des ersten Werkzeugschafts (4) das Drehmoment und die Druckkraft von dem Schrauben-Eingriffsabschnitt (9) des zweiten Werkzeugschafts (5) aufzunehmen, und **dadurch gekennzeichnet, dass** die Schraube einen im Wesentlichen sphärischen oder kegelstumpfförmigen Schraubenkopf hat.

2. Schraubwerkzeug (1) nach Anspruch 1, **dadurch gekennzeichnet, dass** der Schrauben-Eingriffsabschnitt (8, 9) des ersten Werkzeugschafts (4) und/oder des zweiten Werkzeugschafts (5) eine konkav gekrümmte, insbesondere im Wesentlichen sphärische oder kegelstumpfförmige Oberfläche hat.

3. Schraubwerkzeug (1) nach Anspruch 1 oder 2, **gekennzeichnet durch** eine Anziehvorrichtung (12), die dazu vorgesehen und ausgebildet ist, einen Drehwinkel und/oder das Drehmoment des Schrauben-Eingriffsabschnitts (8, 9) des ersten Werkzeugschafts (4) und/oder des zweiten Werkzeugschafts (5) einzustellen und/oder zu messen.

4. Schraubwerkzeug (1) nach einem der Ansprüche 1 bis 3, **gekennzeichnet durch** eine Druckkraftvorrichtung (10), die dazu vorgesehen und ausgebildet ist, die Druckkraft einzustellen, zu berechnen und/oder zu messen.

5. Schraubwerkzeug (1) nach einem der Ansprüche 1 bis 4, **dadurch gekennzeichnet, dass** der erste Werkzeugschaft (4) und der zweite Werkzeugschaft (5) axial zueinander beweglich, beispielsweise schwenkbar oder axialverschieblich, gelagert sind.

6. Schraubwerkzeug (1) nach einem der Ansprüche 1 bis 5, **dadurch gekennzeichnet, dass** der Schrauben-Eingriffsabschnitt (8) des ersten Werkzeugschafts (4) in Axialrichtung zu dem zweiten Werkzeugschaft (5) hin federvorgespannt ist und/oder der Schrauben-Eingriffsabschnitt (9) des zweiten Werkzeugschafts (5) in Axialrichtung zu dem ersten Werkzeugschaft (4) hin federvorgespannt ist.

7. Schraubwerkzeug (1) nach einem der Ansprüche 1 bis 6, **dadurch gekennzeichnet, dass** der erste Werkzeugschaft (4) mit dem zweiten Werkzeugschaft (5) integral um seine Längsachse drehgekoppelt verbunden ist

8. Schraubwerkzeug (1) nach einem der Ansprüche 1 bis 6, **dadurch gekennzeichnet, dass** der erste Werkzeugschaft (4) relativ zu dem zweiten Werkzeugschaft (5) um seine Längsachse relativ drehbar ist.

9. Montageverfahren zum Einschrauben einer Schraube (2) in ein Innengewinde eines medizinischen Instruments (3), mit einem Schraubwerkzeug (1), nach einem der Ansprüche 1 bis 8, um zwei Branchen (20, 21) des Instruments (3) so miteinander zu verbinden, dass die zwei Branchen (20, 21) zueinander verschwenkbar sind, **gekennzeichnet durch** die folgenden Schritte:
- axiales Aufeinander-Zubewegen eines ersten Werkzeugschafts (4) und eines zweiten Werkzeugschafts (5) des Schraubwerkzeugs (1) zum Einspannen der Schraube (2) zwischen ihrem Schraubenkopf (6) und ihrem Schraubenschaft (7) in das Schraubwerkzeug (1), so dass eine Druckkraft axial von dem zweiten Werkzeugschaft (5) in Richtung hin zum ersten Werkzeugschaft (4) auf die Schraube (2) übertragen wird,
- Drehen des Schraubwerkzeugs (1), insbesondere des ersten Werkzeugschafts (4), relativ zu dem Instrument (3) um eine Schraubenlängsachse, um ein Drehmoment, insbesondere zur Einstellung eines Anzugsmoments oder einer Einschraubtiefe der Schraube (2), auf die in das Schraubwerkzeug (1) eingespannte Schraube (2) zu übertragen und die Schraube (2) in das Innengewinde einzuschrauben, und
- axiales Auseinanderbewegen des ersten Werkzeugschafts (4) und des zweiten Werkzeugschafts (5) zum Ausspannen der Schraube (2) aus dem Schraubwerkzeug (1).

10. Montageverfahren nach Anspruch 9, wobei eine erste Branche (20) der beiden Branchen (20, 21) ein erstes Durchgangsloch aufweist und eine zweite Branche (21) der beiden Branchen (20, 21) ein zweites Durchgangsloch aufweist, an dem das Innengewinde ausgebildet ist, **gekennzeichnet durch** den folgenden Schritt:
- axiales Bewegen des zweiten Werkzeugschafts (5) auf den ersten Werkzeugschaft (4) zu und durch das zweite Durchgangsloch hindurch, bevor die Schraube (2) in das Schraubwerkzeug (1) eingespannt sowie in das Innengewinde eingesetzt wird, oder
- Hindurchführen der Schraube (2) durch das erste Durchgangsloch hindurch und Einsetzen der Schraube (2) in das Innengewinde, bevor der zweite Werkzeugschaft (5) durch das zweite Durchgangsloch hindurch auf den ersten Werkzeugschaft (4) axial zubewegt wird und die Schraube (2) in das Schraubwerkzeug (1) eingespannt wird.

11. Montageverfahren nach Anspruch 9 oder 10, bei dem die Schraube (2) in ihrer eingeschraubten Position durch Laserschweißen und/oder durch vor dem Einspannen in das Schraubwerkzeug (1) aufgebrachten Klebstoff gesichert wird.

## Claims

1. A screwdriving tool (1) for screwing a screw (2) in particular into an internal thread of a medical instrument (3), comprising
- a first tool shaft (4) preferably having a large shaft diameter and an end-side screw engagement portion (8) which is provided and configured for transmitting a torque to the screw (2),
- a second tool shaft (5) which preferably has a small shaft diameter and is orientable or oriented axially to the first tool shaft (4) and in the opposite direction thereto such that its end-side screw engagement portion (9) is axially opposite to the end-side screw engagement portion (8) of the first tool shaft (4), and is provided and configured to transmit a compression force axially to the screw (2) in the direction of the first tool shaft (4), and
- a screw provided and configured to receive, from the screw engagement portion (8) of the first tool shaft (4), the torque and the compression force from the screw engagement portion (9) of the second tool shaft (5) and **characterized in that** the screw has a substantially spherical or frustoconical screw head.

2. The screwdriving tool (1) according to claim 1, **characterized in that** the screw engagement portion (8, 9) of the first tool shaft (4) and/or of the second tool shaft (5) have/has a concavely curved surface, in particular a substantially spherical or frustoconical surface.

3. The screwdriving tool (1) according to claim 1 or 2, **characterized by** a tightening device (12) provided and configured to set and/or measure a rotation angle and/or torque of the screw engagement portion (8, 9) of the first tool shaft (4) and/or of the second tool shaft (5).

4. The screwdriving tool (1) according to any of claims 1 to 3, **characterized by** a compression force device (10) which is provided and configured to set, calculate and/or measure the compression force.

5. The screwdriving tool (1) according to any of claims 1 to 4, **characterized in that** the first tool shaft (4) and the second tool shaft (5) are held axially movable, for example, axially pivotable or axially shiftable, relative to each other.

6. The screwdriving tool (1) according to any of claims 1 to 5, **characterized in that** the screw engagement portion (8) of the first tool shaft (4) is spring pre-tensioned in an axial direction toward the second tool shaft (5), and/or the screw engagement portion (9) of the second tool shaft (5) is spring pre-tensioned in the axial direction toward the first tool shaft (4).

7. The screwdriving tool (1) according to any of claims 1 to 6, **characterized in that** the first tool shaft (4) is integrally connected to the second tool shaft (5) in a rotationally coupled manner about its longitudinal axis.

8. The screwdriving tool (1) according to any of claims 1 to 6, **characterized in that** the first tool shaft (4) is relatively rotatable relative to the second tool shaft (5) about its longitudinal axis.

9. An assembly method for screwing a screw (2) into an internal thread of a medical instrument (3), using a screwdriving tool (1) according to any of claims 1 to 8, in order to connect two branches (20, 21) of the instrument (3) to each other such that the two branches (20, 21) are pivotable with respect to each other, **characterized by** the following steps:
- axially moving a first tool shaft (4) and a second tool shaft (5) of the screwdriving tool (1) toward each other for clamping the screw (2) between its screw head (6) and its screw shaft (7) in the screwdriving tool (1), so that a compression force is transmitted axially from the second tool shaft (5) to the screw (2) in the direction toward the first tool shaft (4),
- rotating the screwdriving tool (1), in particular the first tool shaft (4), relative to the instrument (3) about a longitudinal screw axis in order to transmit a torque, in particular for setting a tightening torque or a penetration depth of the screw (2), to the screw (2) clamped in the screwdriving tool (1), and to screw the screw (2) into the internal thread, and
- axially moving the first tool shaft (4) and the second tool shaft (5) apart to unclamp the screw (2) from the screwdriving tool (1).

10. The assembly method of claim 9, wherein a first branch (20) of the two branches (20, 21) comprises a first through-hole and a second branch (21) of the two branches (20, 21) comprises a second through-hole in which the internal thread is formed, **characterized by** the following step:
- axially moving the second tool shaft (5) toward the first tool shaft (4) and through the second through-hole before the screw (2) is clamped into the screwdriving tool (1) and inserted into the internal thread, or
- passing the screw (2) through the first through-hole and inserting the screw (2) into the internal thread before moving the second tool shaft (5) axially through the second through-hole toward the first tool shaft (4) and clamping the screw (2) into the screwdriving tool (1).

11. The assembly method according to claim 9 or 10, in which the screw (2) is secured in its screwed-in position by laser welding and/or by adhesive applied before clamping in the screwdriving tool (1).

## Revendications

1. Outil de vissage (1) destiné à visser une vis (2), en particulier dans un filetage intérieur d'un instrument médical (3), avec
- une première tige d'outil (4) qui présente de préférence un grand diamètre de tige et qui présente une section d'engagement de vis (8) du côté de l'extrémité, qui est prévue et configurée pour transmettre un couple de rotation à la vis (2),
- une seconde tige d'outil (5) qui présente de préférence un petit diamètre de tige et qui peut être orientée ou est orientée axialement par rapport à la première tige d'outil (4) ainsi qu'en sens inverse de celle-ci, de telle sorte que sa section d'engagement de vis (9) du côté de l'extrémité est axialement opposée à la section d'engagement de vis (8) du côté de l'extrémité de la première tige d'outil (4) et est prévue et configurée pour transmettre à la vis (2) une force de pression axialement en direction de la première tige d'outil (4), et
- une vis qui est prévue et configurée pour recevoir de la partie d'engagement de vis (8) de la première tige d'outil (4) le couple de rotation et la force de compression de la partie d'engagement de vis (9) de la seconde tige d'outil (5) et **caractérisé en ce que** la vis présente une tête de vis sensiblement sphérique ou tronconique.

2. Outil de vissage (1) selon la revendication 1, **caractérisé en ce que** la section d'engagement de vis (8, 9) de la première tige d'outil (4) et/ou de la seconde tige d'outil (5) présente une surface incurvée concave, en particulier sensiblement sphérique ou tronconique.

3. Outil de vissage (1) selon la revendication 1 ou 2, **caractérisé par** un dispositif de serrage (12) qui est prévu et configuré pour régler et/ou mesurer un angle de rotation et/ou le couple de rotation de la section d'engagement de vis (8, 9) de la première tige d'outil (4) et/ou de la seconde tige d'outil (5).

4. Outil de vissage (1) selon l'une quelconque des revendications 1 à 3, **caractérisé par** un dispositif de force de compression (10) qui est prévu et configuré pour régler, calculer et/ou mesurer la force de compression.

5. Outil de vissage (1) selon l'une quelconque des revendications 1 à 4, **caractérisé en ce que** la première tige d'outil (4) et la seconde tige d'outil (5) sont montées mobiles axialement l'une par rapport à l'autre, par exemple de manière pivotante ou déplaçable axialement.

6. Outil de vissage (1) selon l'une quelconque des revendications 1 à 5, **caractérisé en ce que** la partie d'engagement de vis (8) de la première tige d'outil (4) est précontrainte par ressort dans la direction axiale vers la seconde tige d'outil (5) et/ou la partie d'engagement de vis (9) de la seconde tige d'outil (5) est précontrainte par ressort dans la direction axiale vers la première tige d'outil (4).

7. Outil de vissage (1) selon l'une quelconque des revendications 1 à 6, **caractérisé en ce que** la première tige d'outil (4) est reliée à la seconde tige d'outil (5) de manière intégrale et couplée en rotation autour de son axe longitudinal.

8. Outil de vissage (1) selon l'une quelconque des revendications 1 à 6, **caractérisé en ce que** la première tige d'outil (4) peut tourner relativement par rapport à la seconde tige d'outil (5) autour de son axe longitudinal.

9. Procédé de montage pour visser une vis (2) dans un filetage interne d'un instrument médical (3), avec un outil de vissage (1), selon l'une quelconque des revendications 1 à 8, pour relier deux branches (20, 21) de l'instrument (3) de telle sorte que les deux branches (20, 21) puissent pivoter l'une par rapport à l'autre, **caractérisé par** les étapes suivantes :
- le rapprochement axial d'une première tige d'outil (4) et d'une seconde tige d'outil (5) de l'outil de vissage (1) l'une vers l'autre pour serrer la vis (2) entre sa tête de vis (6) et sa tige de vis (7) dans l'outil de vissage (1), de sorte qu'une force de compression soit transmise axialement à la vis (2) de la seconde tige d'outil (5) en direction de la première tige d'outil (4),
- la rotation de l'outil de vissage (1), en particulier de la première tige d'outil (4), par rapport à l'instrument (3) autour d'un axe longitudinal de vis, afin de transmettre à la vis (2) serrée dans l'outil de vissage (1) un couple de rotation, en particulier pour régler un couple de serrage ou une profondeur de vissage de la vis (2), et de visser la vis (2) dans le filetage intérieur, et
- l'écartement axial de la première tige d'outil (4) et de la seconde tige d'outil (5) pour desserrer la vis (2) de l'outil de vissage (1).

10. Procédé de montage selon la revendication 9, dans lequel une première branche (20) des deux branches (20, 21) présente un premier trou traversant et une seconde branche (21) des deux branches (20, 21) présente un second trou traversant au niveau duquel est formé le filetage interne, **caractérisé par** l'étape suivante :
- le déplacement axial de la seconde tige d'outil (5) vers la première tige d'outil (4) et à travers le second trou traversant, avant que la vis (2) soit serrée dans l'outil de vissage (1) et insérée dans le filetage intérieur, ou
- le passage de la vis (2) à travers le premier trou traversant et l'insertion de la vis (2) dans le filetage intérieur, avant que la seconde tige d'outil (5) soit déplacée axialement vers la première tige d'outil (4) à travers le second trou traversant et que la vis (2) soit serrée dans l'outil de vissage (1).

11. Procédé de montage selon la revendication 9 ou 10, dans lequel la vis (2) est bloquée dans sa position vissée par soudage au laser et/ou par de la colle appliquée avant le serrage dans l'outil de vissage (1).
